Europäisches Patentamt

European Patent Office

Office européen des brevets

(11) Publication number: **0 502 607 A1**

## EUROPEAN PATENT APPLICATION

(21) Application number: **92300671.2**

(22) Date of filing: **27.01.92**

(51) Int. Cl.⁵: **C12N 15/16**, C07K 15/00

A request for correction of claim 9 and 10 has been filed pursuant to Rule 88 EPC. A decision on the request will be taken during the proceedings before the Examining Division (Guidelines for Examination in the EPO, A-V, 2.2).

(30) Priority: **11.02.91 IL 97212**

(43) Date of publication of application:
**09.09.92 Bulletin  92/37**

(84) Designated Contracting States:
**AT BE CH DE DK ES FR GB GR IT LI LU MC NL PT SE**

(71) Applicant: **BAR ILAN UNIVERSITY**
**P.O. Box 1530**
**IL-52100 Ramat-Gan(IL)**

(72) Inventor: **Shoham, Jacob**
**71 Mandes Street**
**Ramat Gan 52653(IL)**
Inventor: **Sonkin-Zevin, Dina**
**25 Ezra Street**
**Rehovot 76201(IL)**

(74) Representative: **Hillier, Peter et al**
**Reginald W. Barker & Co., 13, Charterhouse Square**
**London, EC1M 6BA(GB)**

(54) **Thymopoietin.**

(57) There are described bovine thymopoietin or thymopoietin-like cDNA clones containing a sequence corresponding to at least part of bovine thymopoietin mRNA by virtue of the fact that it includes at least the sequential nucleotides in the following structure corresponding to amino acids 18 to 49:

|  1  |  2  |  3  |  4  |  5  |  6  |  7  |  8  |  9  | 10  | 11  | 12  | 13  | 14  | 15  | 16  |
|-----|-----|-----|-----|-----|-----|-----|-----|-----|-----|-----|-----|-----|-----|-----|-----|
| CCG | GAG | TTC | CTG | GAA | GAC | CCC | TCG | GTC | CTG | ACG | AAA | GAG | AAG | TTG | AAG |

| 17  | 18  | 19  | 20  | 21  | 22  | 23  | 24  | 25  | 26  | 27  | 28  | 29  | 30  | 31  | 32  |
|-----|-----|-----|-----|-----|-----|-----|-----|-----|-----|-----|-----|-----|-----|-----|-----|
| AGT | GAG | TTG | GTC | GCC | AAC | AAT | GTG | ACG | CTC | CCG | GCC | GGG | GAG | CAG | CGC |

| 33  | 34  | 35  | 36  | 37  | 38  | 39  | 40  | 41  | 42  | 43  | 44  | 45  | 46  | 47  | 48  |
|-----|-----|-----|-----|-----|-----|-----|-----|-----|-----|-----|-----|-----|-----|-----|-----|
| AAA | GAC | GTG | TAT | GTG | CAG | CTC | TAC | CTG | CAG | CAC | CTC | ACG | GCG | CGC | AAC |

| 49  |
|-----|
| CGG. |

Examples are given of such cDNA clones contain either the whole of the nucleotide sequence corresponding to amino acids 1 to 49, or a nucleotide sequence corresponding to amino acids 18 to 49. Further aspects are a bovine thymopoietin mRNA species having a nucleotide sequence corresponding to the nucleotide sequence of the cDNA clones of the invention; the deduced protein sequence according to the translatable part of these mRNA species (and bioactive cleavage fragments deduced therefrom); isolated and purified DNA clones which contain bovine thymopoietin gene sequence and include the sequence corresponding to amino acids 1 to 44;as well as cDNA and DNA produced by hybridization from a cDNA clone or an isolated and purified DNA clone according to the invention.

```
                  15                      30                      45
C    TGC AGG GGC TTT TGT GTC CGC CTT TGG CTT GGC TTT GTG TGC GCG GGT TTC


             60                      75                      90                     105
CGC TGG GCT TCC AGG CGT CAG TCC CGG GCG GGA GCC GTG AGG CTC GGA GGC GGG


                 120                     135                     150
AGC GCG GCC CCC CCT CCC CCG GGC GAG GAG CGA GCG CGC CGG CGT GAG CGG AGG


    165                     180                     195                     210
GTG TGA GGG CTG CGG GGA AGG GGA TTC GCC GCC GAG ATG CCG GAG TTC CTG GAA
     *      Gly Leu Arg Gly Arg Gly Phe Ala Ala Glu Met Pro Glu Phe Leu Glu
                                                         1


                 225                     240                     255
GAC CCC TCG GTC CTG ACG AAA GAG AAG TTG AAG AGT GAG TTG GTC GCC AAC AAT
Asp Pro Ser Val Leu Thr Lys Glu Lys Leu Lys Ser Glu Leu Val Ala Asn Asn
                      11                                          21

270                     285                     300                     315
GTG ACG CTC CCG GCC GGG GAG CAG CGC AAA GAC GTG TAT GTG CAG CTC TAC CTG
Val Thr Leu Pro Ala Gly Glu Gln Arg Lys Asp Val Tyr Val Gln Leu Tyr Leu
                           31                                          41

CAG
Gln
```

Figure 3

FIELD AND BACKGROUND OF THE INVENTION

The present invention relates to bovine thymopoietin cDNA clone, fetal calf thymopoietin cDNA clone, fetal calf thymopoietin-like cDNA clone, a bovine thymopoietin mRNA, fetal calf thymopoietin mRNA, fetal calf thymopoietin-like mRNA, the deduced protein sequence according to the translatable part of these mRNAs, and bioactive cleavage fragments deduced therefrom, as well as isolated DNA clones which contain bovine thymopoietin gene sequence and cDNA or DNA clones produced by hybridization from the clones of the invention.

The thymus is a central organ of the immune system, which mediates a major part of its activity by secreting several humoral factors. One of these factors, named thymopoietin (TP), was isolated from calf thymus (Goldstein, G., Nature, 1974 247: 11-14). The sequence of TP was first described as follows (Schlesinger, D.H. and Goldstein G., Cell, 1975 5: 361-5):

$$NH_2\text{-Ser-Gln-Phe-Leu-Glu(5)-Asp-Pro-Ser-Val-Leu(10)-}$$

$$Thr\text{-Lys-Glu-Lys-Leu(15)-Lys-Ser-Glu-Leu-Val(20)-}$$

$$Ala\text{-Asn-Asn-Val-Thr(25)-Leu-Pro-Ala-Gly-Glu(30)-}$$

$$Gln\text{-Arg-Lys-Asp-Val(35)-Tyr-Val-Gln-Leu-Tyr(40)-}$$

$$Leu\text{-Gln-Thr-Leu-Thr(45)-Ala-Val-Lys-Arg(49)-COOH.}$$

Subsequent work (see e.g. Audhya, T., Schlesinger, D.H. and Goldstein, G., Biochemistry, 1981, 20: 6195-6200) disclosed the following three molecular species, termed TP-I, TP-II and TP-III, respectively, which as will be seen differ structurally in a restricted number of amino acids:

(TP-I:)

NH$_2$-GIy-Gln-Phe-Leu-Glu(5)-Asp-Pro-Ser-Val-Leu(10)-

Thr-Lys-Glu-Lys-Leu(15)-Lys-Ser-Glu-Leu-Val(20)-

Ala-Asn-Asn-Val-Thr(25)-Leu-Pro-Ala-Gly-Glu(30)-

Gln-Arg-Lys-Asp-Val(35)-Tyr-Val-Glu-Leu-Tyr(40)-

Leu-Gln-His-Leu-Thr(45)-Ala-Leu-Lys-Arg(49)-COOH.

(TP-II:)

NH$_2$-Pro-Glu-Phe-Leu-Glu(5)-Asp-Pro-Ser-Val-Leu(10)-

Thr-Lys-Glu-Lys-Leu(15)-Lys-Ser-Glu-Leu-Val(20)-

Ala-Asn-Asn-Val-Thr(25)-Leu-Pro-Ala-Gly-Glu(30)-

Gln-Arg-Lys-Asp-Val(35)-Tyr-Val-Glu-Leu-Tyr(40)-

Leu-Gln-Ser-Leu-Thr(45)-Ala-Leu-Lys-Arg(49)-COOH.

(TP-III:)

NH$_2$-Pro-Glu-Phe-Leu-Glu(5)-Asp-Pro-Ser-Val-Leu(10)-

Thr-Lys-Glu-Lys-Leu(15)-Lys-Ser-Glu-Leu-Val(20)-

Ala-Asn-Asn-Val-Thr(25)-Leu-Pro-Ala-Gly-Glu(30)-

Gln-Arg-Lys-Glu-Val(35)-Tyr-Val-Glu-Leu-Tyr(40)-

Leu-Gln-His-Leu-Thr(45)-Ala-Leu-Lys-Arg(49)-COOH.

Goldstein, G. et al in Science, 1979 204: 1309-11, found that the active site resided in the amino acid sequence 32-36, and synthesized this pentapeptide, termed thymopentin, the in vitro activity of which was similar to that of the whole molecule. TP-III, which contains glutamic acid instead of aspartic acid in position 34, is formed also in the spleen (Audhya, T., Scheid, M.P. and Goldstein, G., P.N.A.S. 1984 81: 2847-9), and is termed splenin. Splenin has a nonspecific effect on lymphoid cells, i.e. it induces differentiation surface markers in both T and B cells, whereas thymopoietins I and II are specific to T cells. Therefore, TP-III, although similar in structure to the other molecular species detailed above, is not regarded as a true thymic factor.

Thymopoietin and thymopentin have an important influence on the immune system, and in particular appear to have beneficial clinical effects in conditions of immunodeficiency, cancer, infectious diseases and autoimmune disorders. On the other hand, no significant adverse reactions to thymic factors have been documented. Thus, these factors represent a therapeutic approach which is based on normal physiological mechanism, presents a broad spectrum of potential clinical use and is devoid of undesirable side effects.

There are, however, a number of drawbacks to the practical utilization of thymic factors. The preparation of thymopoietin for general clinical use, either by extraction and purification from natural sources, or by chemical synthesis, would be uneconomical. Also, while thymopentin can be chemically synthesized economically and in viable quantities, it has been shown to have an extremely short half-life, when injected in vivo. Therefore, it seems that the whole thymopoietin molecule is necessary for stability, and it would be most desirable to use this agent for treatment, if it were to be available.

It is an object of the present invention to provide means by which thymopoietin may be obtained economically. Other objects of the invention will appear from the description which follows.

SUMMARY OF THE INVENTION

The invention accordingly provides a bovine thymopoietin or thymopoietin-like cDNA clone which contains a sequence corresponding to at least part of bovine thymopoietin mRNA by virtue of the fact that it includes at least the sequential nucleotides in the following structure corresponding to amino acids 18 to 49:

```
  1   2   3   4   5   6   7   8   9  10  11  12  13  14  15  16
CCG GAG TTC CTG GAA GAC CCC TCG GTC CTG ACG AAA GAG AAG TTG AAG

 17  18  19  20  21  22  23  24  25  26  27  28  29  30  31  32
AGT GAG TTG GTC GCC AAC AAT GTG ACG CTC CCG GCC GGG GAG CAG CGC

 33  34  35  36  37  38  39  40  41  42  43  44  45  46  47  48
AAA GAC GTG TAT GTG CAG CTC TAC CTG CAG CAC CTC ACG GCG CGC AAC

 49
CGG.
```

Such a cDNA clone may include the whole of the nucleotide sequence corresponding to amino acids 1 to 49 recited above, such as a bovine thymopoietin cDNA clone in which said nucleotide sequence corresponding to amino acids 1 to 49 is included in an open reading frame of 741 nucleotides, or a partial bovine fetal thymopoietin cDNA clone in which said nucleotide sequence corresponding to amino acids 1 to 49 is included in an open reading frame of 323 nucleotides, and wherein a 45 nucleotide sequence at the 3'-end of said open reading frame is different from that in the cDNA clone of the just-mentioned bovine thymopoietin cDNA clone.

In a different embodiment of the invention, the cDNA clone may be e.g. a bovine fetal thymopoietin-like cDNA clone, which contains an open reading frame of 537 nucleotides corresponding to a 179 amino acid polypeptide of which the N-terminal part includes amino acids 18 to 49 of thymopoietin.

The invention also provides a bovine thymopoietin mRNA species having a nucleotide sequence corresponding to the nucleotide sequence of the cDNA clone of the invention as defined herein. Also provided by the invention are the deduced protein sequence according to the translatable part of these mRNA species, and any bioactive cleavage fragment deduced therefrom.

Further provided by the invention are isolated and purified DNA clones which contain bovine thymopoietin gene sequence and include the sequence:

```
  1   2   3   4   5   6   7   8   9  10  11  12  13  14  15  16
CCG GAG TTC CTG GAA GAC CCC TCG GTC CTG ACG AAA GAG AAG TTG AAG

 17  18  19  20  21  22  23  24  25  26  27  28  29  30  31  32
AGT GAG TTG GTC GCC AAC AAT GTG ACG CTC CCG GCC GGG GAG CAG CGC

 33  34  35  36  37  38  39  40  41  42  43  44
AAA GAC GTG TAT GTG CAG CTC TAC CTG CAG CAC CTC.
```

Yet further provided by the invention are cDNA and DNA which are produced by hybridization from a cDNA clone or an isolated and purified DNA clone according to the invention as defined in the foregoing paragraphs.

BRIEF DESCRIPTION OF THE DRAWINGS

Figure 1A shows a UV photograph of the electrophoresed restriction digests of two bovine genomic DNA clones;

Figure 1B shows an autoradiogram of the restriction fragments of two bovine genomic DNA clones after hybridization to radioactive TP-CDi;

Figure 2 shows restriction maps of two isolated bovine genomic DNA clones;

Figure 3 shows a DNA sequence of a fragment of bovine thymopoietin genomic clone and the deduced protein sequence;

Figure 4 shows a restriction map and sequencing strategy scheme of a bovine thymopoietin cDNA clone;

Figure 5 shows a DNA sequence of a bovine thymopoietin cDNA clone and the deduced amino acid sequence of thymopoietin precursor;

Figure 6 shows a DNA sequence of a bovine fetal thymopoietin cDNA clone and the deduced amino acid sequence of a part of fetal bovine thymopoietin precursor;

Figure 7 shows a DNA sequence of a bovine fetal thymopoietin-like cDNA clone and the deduced amino acid sequence of the corresponding protein.

## DETAILED DESCRIPTION OF THE INVENTION

### Hybridization with Oligonucleotide Probes

Oligonucleotide sequences coding for different parts of the thymopoietin peptides and set out in Table I were synthesized for the inventors, using an automatic DNA synthesizer, by Dr. O. Goldberg of the Weizmann Institute, Rehovot. Each sequence was a mixture of alternatives indicated by the symbols { / }. These synthetic oligonucleotides can be used as hybridization probes in searching for the gene coding for the corresponding protein. In order to overcome problems arising from degeneracy of the genetic code, deoxyinosine (I) was inserted in each position where four nucleotides are allowed by codon ambiguity.

```
                Table I: Oligonucleotide Probes.
        -----------------------------------------------------------
        NAME                          PROBE                   LENGTH
                        (begins at 5', terminates at 3')      (NO.OF
                                                              BASES)
        -----------------------------------------------------------


        1TP-ABi*  TC{T/C}TTIGTIA{A/G}IAC{IGA/GCT}IGGGTC{T/C}TC       26


        2TP-ABi♦  GAGGA{C/T}CCI{TCI/ACC/ACT}GTI{C/T}TIACIAA{A/G}GA   26


        3TP-CDi*  AC{A/G}TAIACITC{C/T}TTIC{GC/TT}TG{C/T}TCICCIGCIGG  32


        4TP-DEi*  TGIA{A/G}GTAIA{AC/GT}TCIACGTAIAC                   23


        -----------------------------------------------------------
        *cDNA strand         ♦m-RNA strand
        1) codes for amino acids 5-13; extent of degeneracy 16-fold
        2) codes for amino acids 5-13; extent of degeneracy 24-fold
        3) codes for amino acids 27-37; extent of degeneracy 16-fold
        4) codes for amino acids 35-42; extent of degeneracy 8-fold
```

A genomic DNA library (a kind gift of Dr. B. Vellan), prepared from liver of a Holstein Friesian cow by cloning of DNA partially digested with Sau 3A in the BamH I site of the lambda 47.1L vector, was plated out ($1 \times 10^6$ plaque-forming units) using E. coli LE392 cells on 20 petri dishes (diam. 15 cm.), and nitrocellulose filter copies were prepared and screened with TP-CDi oligonucleotide probe. The hybridization reaction mixture contained 0.9M NaCl, 0.09M trisodium citrate. 0.1% BSA, 0.1% Ficoll, 0.1% polyvinylpyrrolidone, 2mM EDTA, 100 $\mu$g./ml. Herring denatured DNA and $5 \times 10^6$ dpm/ml. of $^{32}$P-end-labelled TP-CDi (specific activity $1 \times 10^7$ dpm/pmole), and was shaken and then incubated for 16 hours at 42°C. The filters were washed several times with a mixture of 0.45M NaCl and 0.045M trisodium citrate at 42°C over a period of 6 hours, followed by 2 x 3M $Me_4N + Cl-$, as described by Wood et al (P.N.A.S. 1977 74: 5463-7). 12 positive clones were isolated after two additional rounds of enrichment plating. DNA was isolated from these clones and hybridization attempted with TP-CDi, TP-ABicDNA or TP-DEi oligonucleotides. None of the clones hybridized with TP-DEi and two clones (152B and 157A) hybridized with both TP-CDi and TP-ABi probes.

6

Clones 152B and 157A were subjected to further characterization.

Restriction Analysis of Hybridizable Clones 152B and 157A

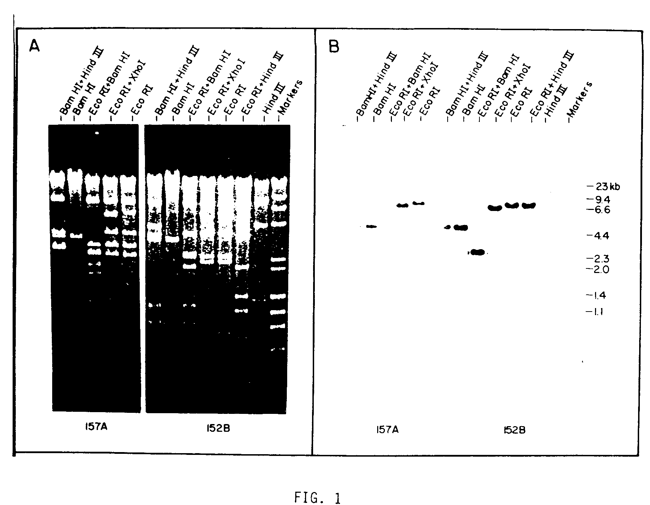

Isolated DNA from these clones was digested with EcoR I, Bam HI, Hind III, Xho I and their combinations. Restrictive fragments were separated by agarose gel electrophoresis, transferred to DNA binding nylon and hybridized with ³²P end-labelled TP-CDi. The results are shown in Figure 1 (A and B).

Figure 1A is a UV photograph of the electrophoresed restriction digests of 152B and 157A, of which 2 µg. (each) were digested with EcoR I, BamH I, Hind III, Xho I and their indicated combinations. Restriction fragments were separated on 1% agarose gel containing 1 µg. per ml. Ethidium Bromide in Tris-borate buffer (see Maniatis et al, Molecular Cloning: A Laboratory Manual, Cold Spring Harbor Laboratory, Cold Spring Harbor, N.Y., U.S.A., 1982) with lambda-Hind III and φX 174-Hae III fragments as size markers. The positions of relevant enzyme sites on the BamH I arms of the vector lambda 47.1L are as follows:

```
Left arm: EcoR I      21.7 kb        Right arm: Hind III   2.24 kb

          Hind III   23   kb                    EcoR I     2.83 kb

overall length        23.6 kb        overall length       10.43 kb
```

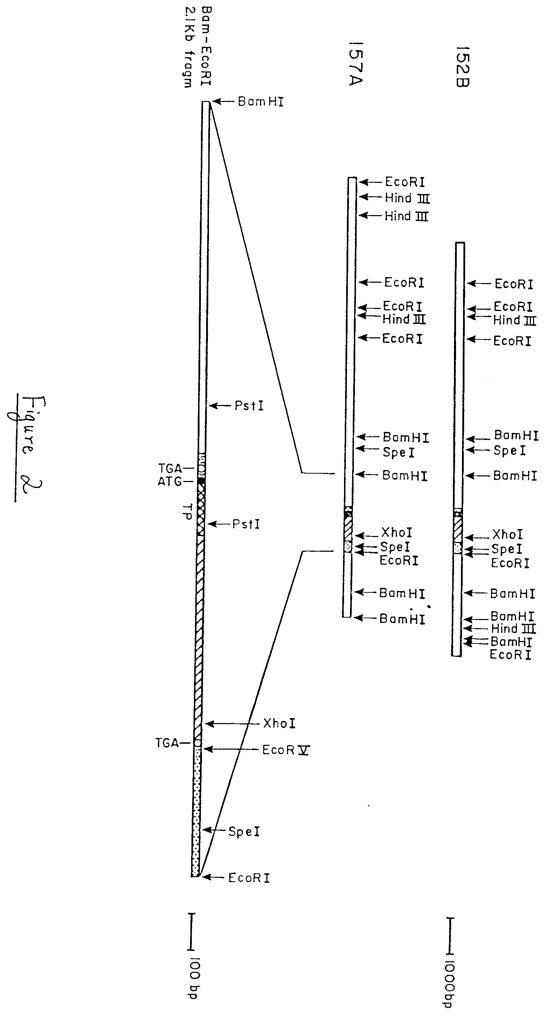

Figure 1B is an autoradiogram of 152B and 157A fragments hybridized with ³²P-labelled TP-CDi. The DNA fragments shown in Fig. 1A were transferred to the NEN Gene Screen membrane in 1N NaOH and the membrane was air dried. DNA was linked to the nylon by exposure to UV on a transilluminator table for 5 minutes, neutralized in 0.5M Tris at pH 7.4 with 1.5M NaCl and hybridized with ³²P-labelled TP-CDi. The membrane was washed with a mixture of 0.45M NaCl, 0.045M trisodium citrate and 0.5% SDS at 50ºC and exposed to AGFA RP-2 film with an X-ray intensifying screen at -70ºC for 3 hours. Additional restriction enzymes, both alone and in combinations, were used for DNA digestion, and the resulting restriction map is shown in Fig. 2.

The results show that (a) the overall length of insert DNA as estimated by summation of the lengths of relevant fragments is about 11 kb for 152B and about 12 kb for 157A; (b) fragments which hybridize to TP-CDi are of identical size in both 152B and 157A, namely a ca. 6 kb EcoR I fragment, a ca. 8.5 kb Hind III fragment, a 3.2 kb BamH I fragment and a 2.1 kb fragment obtained by double EcoR I and BamH I digestion; (c) the two genomic clones represent the same gene which was subcloned at different restriction sites. The 2.1 kb fragment (of 152B) was isolated and cut further with one of Alu I, Hae III or Pst I, in order to find fragments free of repetitive DNA sequences (e.g. the Alu family and the like). A 300 b.p. fragment obtained by digestion with Pst I hybridized to TP-CDi was apparently devoid of repetitive DNA and was thus suitable to serve as a probe for a cDNA clone search. This Pst I fragment was subcloned in multipurpose plasmid vector Bluescript SKM13 (+) (Stratagene), which contains T3 and T7 promoters and can be grown as single-stranded M13-like phage for DNA sequencing by the chain-termination method.

DNA sequence of part of 152B and 157A

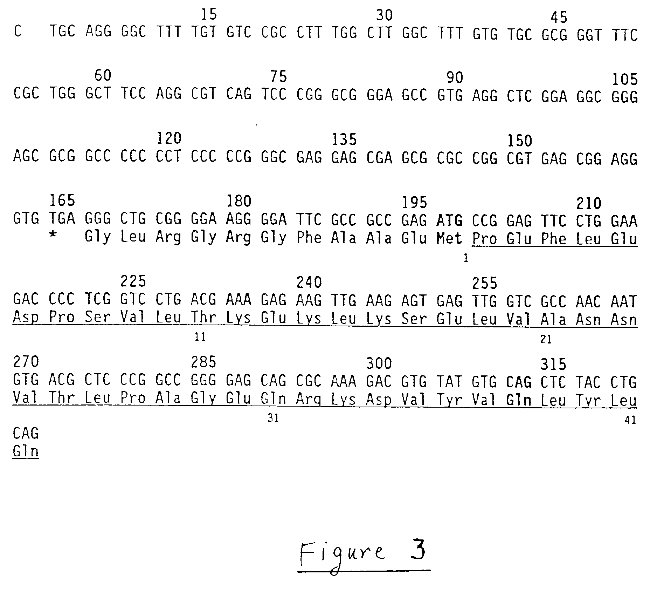

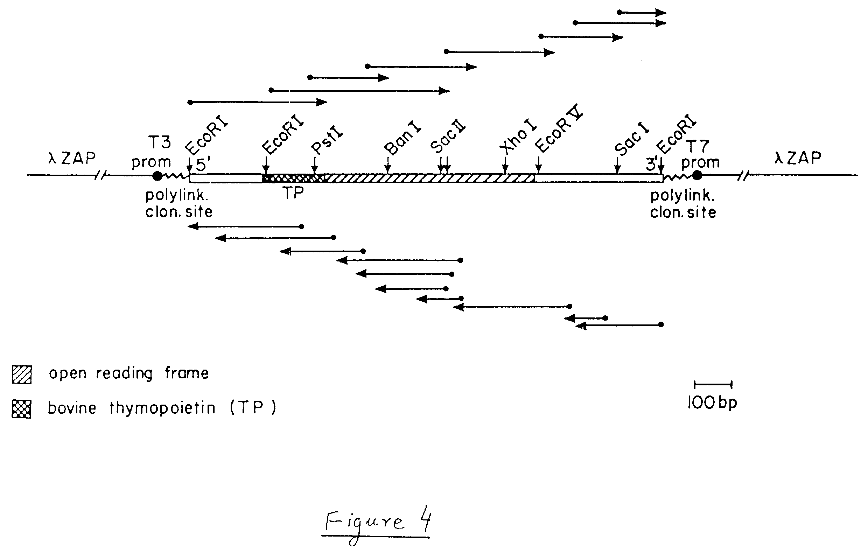

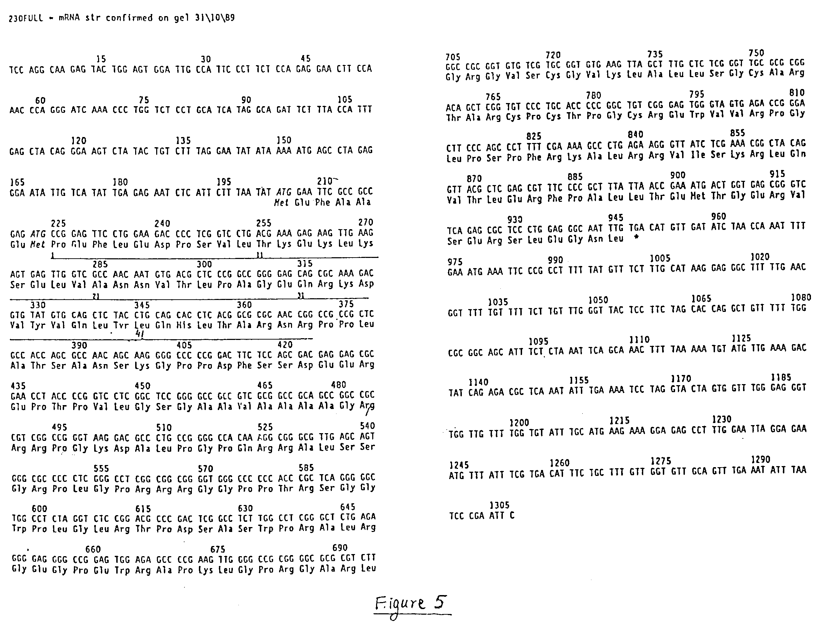

DNA sequencing analysis (for amino acids 22-44) of the double-stranded DNA insert at both 152B and 157A was performed by the dideoxy chain termination method of Sanger et al (P.N.A.S. 1977, 74: 5463-7) using reverse transcriptase (Zagursky et al, Gene and Tech 1985, 2: 89-94) and TP-ABi mRNA oligonucleotide as primer. Single-stranded DNA from recombinant Bluescript SKM13 (+) subclone of 325 bp Pst I fragment of 152B clone (152B-13) was sequenced using Klenow fragment of Pol I and T7 promoter-primer (NE Biolabs No. 1227). The DNA sequence and the deduced peptide sequence are shown in Fig. 3. The sequence coding for amino acids 1-42 of thymopoietin is underlined; methionine which may serve as initiator amino acid is bold-face and an asterisk indicates termination codon. The sequence coding for carboxy-terminal 7 amino acids was cut out from this clone by Pst I digestion. The thymopoietin peptide sequence deduced from clone 152-13, as compared with the published amino acid sequence of thymopoietin is as follows:

### DNA sequence of part of 152B bovine genomic DNA clone.

```
                  <--------- TP-ABi mRNA primer---->
                  |                              |
(1) CCG GAG TTC CTG GAA GAC CCC TCG GTC CTG ACG AAA GAG AAG TTG AAG AGT GAG TTG GTC GCC AAC AAT GTG ACG
(2) Pro-Glu-Phe-Leu-Glu-Asp-Pro-Ser-Val-Leu-Thr-Lys-Glu-Lys-Leu-Lys-Ser-Glu-Leu-Val-Ala-Asn-Asn-Val-Thr-
(3) Pro-Glu-Phe-Leu-Glu-Asp-Pro-Ser-Val-Leu-Thr-Lys-Glu-Lys-Leu-Lys-Ser-Glu-Leu-Val-Ala-Asn-Asn-Val-Thr-
(4) Gly-Gln-Phe-Leu-Glu-Asp-Pro-Ser-Val-Leu-Thr-Lys-Glu-Lys-Leu-Lys-Ser-Glu-Leu-Val-Ala-Asn-Asn-Val-Thr-
```

```
                                                    <Pst I>
                                                    |     |
(1) CTC CCG GCC GGG GAG CAG CGC AAA GAC GTG TAT GTG CAG  CTC TAC CTG CAG CAG CTC
(2) Leu-Pro-Ala-Gly-Glu-Gln-Arg-Lys-Asp-Val-Tyr-Val-Gln -Leu-Tyr-Leu-Gln-His-Leu
(3) Leu-Pro-Ala-Gly-Glu-Gln-Arg-Lys-Asp-Val-Tyr-Val-Gln*-Leu-Tyr-Leu-Gln-Ser-Leu-Thr-Ala-Leu-Lys-Arg
(4) Leu-Pro-Ala-Gly-Glu-Gln-Arg-Lys-Asp-Val-Tyr-Val-Gln -Leu-Tyr-Leu-Gln-His-Leu-Thr-Ala-Leu-Lys-Arg
```

KEY: (1) DNA Sequence 152B; (2) Deduced AA Sequence 152B;
        (3) Published AA Sequence TP-II; (4) Published AA Sequence TP- I.

The sequenced part of 152B DNA is identical to the sequenced part of 157A DNA (amino acids 22-44) and codes for a peptide identical with the first 44 amino acids of TP II except for position 43 which is histidine (as in TP I) instead of serine. Position 38 in our sequence is glutamine and not glutamic acid as described in some of the prior art.

### Preparation of cDNA library from calf thymic epithelial mRNA

A full-length DNA clone coding for thymopoietin(s) is necessary in order to study the structure of the mRNA and to examine whether there are active precursor(s) and/or other thymic-stimulating factor(s) on the same mRNA. For this purpose, a cDNA library starting from calf thymus enriched for epithelial cells was prepared. Thymus from 4-month old calves was ground over a fine sieve and the majority of thymocytes were removed by being allowed to pass through. The remaining tissue was used for RNA extraction using the guanidine thiocyanate-urea/LiCl method (Cathala et al, DNA, 1983, 2: 329-335). mRNA was isolated by twice binding to a column of oligo-dT cellulose. Double-stranded complementary DNA was synthesized using an Amersham cDNA synthesis kit in the following manner. The first strand of the cDNA was synthesized on an mRNA template with the enzyme reverse transcriptase and oligo-dT as primer. The second strand was synthesized with E. coli polymerase I utilizing RNA fragments generated by digestion of mRNA with RNase H as primers. Double-stranded cDNA was ligated into EcoR I site of lambda-ZAP phage DNA (Stratagene) using EcoR I linkers. The average size of double-stranded cDNA was ca. 700 bp as estimated by agarose gel electrophoresis (majority of the DNA migrating in the 300-1200 bp range). Double-stranded lambda-ZAP DNA was packaged using Stratagene Giga-Gold in vitro packaging extracts and plated with E. coli BB4 bacteria. The overall yield was $4.6 \times 10^6$ independent recombinants from 2 $\mu$g. of polyadenylated RNA. This library was amplified once on agar plates under conditions such that there are still isolated plaques, in order to minimize competition and selection between individual recombinants.

### Isolation and characterization of calf cDNA clone coding for thymopoietin

Calf thymic epithelial cDNA library ($1 \times 10^6$ plaque-forming units) was plated out using E. coli BB4, and its nitrocellulose replica filters copies were hybridized with 32P-labelled 325 bp Pst I genomic DNA fragment (isolated from clone 152B-13). The hybridization was performed overnight at 65°C in 0.9M NaCl, 0.09M trisodium citrate. 0.1% BSA, 0.1% Ficoll, 0.1% polyvinylpyrrolidon, 2mM EDTA, 0.5% SDS, 100 $\mu$g./ml. Herring denatured DNA and $5 \times 10^6$ dpm/ml. of $^{32}$P-labelled DNA probe (specific activity $7.5 \times 10^8$ dpm/$\mu$g.). The DNA was labelled by the random primer extension method (Feinberg et al, Anal. Biochem. 1983, 132: 6-13). The filters were washed with a mixture of 15 mM NaCl and 1.5 mM trisodium citrate at 50°C. Only one clone (No. 230) of $10^6$ was positive. This clone was purified by two rounds of enrichment plating followed by hybridization to the probe. The lambda-ZAP phage, which was used as a vector for cloning of the cDNA library, has the advantage of being convertible, depending on the conditions of growth, either to a single-stranded Bluescript SKM13 (-) phage or to Bluescript SKM13 (-) plasmid, thus enabling rapid characterization of the recombinant clone by restriction analysis and DNA sequencing.

DNA Sequence of the 230 Clone

DNA was isolated from clone 230 and converted to Bluescript SKM13(-) plasmid. Series of overlapping deletion subclones were derived by limited digestion of 230 DNA with Exo III exonuclease starting from BstX site in the polylinker of Bluescript, according to the manual of the stratagene Bluescript Exo/Mung kit (Stratagene, No. 200300). In order to obtain the sequence of the opposite strand, the entire insert was subcloned in Bluescript SKM13(+) vector, and its deletion subclones starting from KpnI site in polylinker were produced likewise. The single-stranded DNA was sequenced by the dideoxy chain termination method (Sanger, F. et al, PNAS, 1977, 74: 5963-7) using either T7 DNA polymerase (Sequenase kit, USB No. 70700) or Taq DNA polymerase (Taq Track, promega No. Q5560) starting from T7 promoter primer for SKM13(+) and T3 promoter primer for SKM13(-) DNA. The scheme representing a restriction map and sequencing strategy of clone 230 is given in Fig. 4, in which arrows represent the direction and length of the sequenced regions; location of the open reading frame and thymopoietin sequence are also indicated.

Fig. 5 shows the 1306 bp nucleotide sequence of 230 insert DNA which was obtained and confirmed as described with respect to Fig. 2. Nucleotides are numbered in the 5'- to 3'-direction, and the predicted amino acid sequence is shown below the corresponding nucleotide sequence. Continuously underlined are 49 amino acids corresponding to bovine thymopoietin II, while bold type indicates amino acids which either differ from thymopoietin (Arg 47 and Asn 48), or match other thymopoietins (His 43 as in thymopoietin I and III), or is controversial in the literature (Gln 38 is reported as Glu in some publications). Italics indicate Met which can serve as translation initiator and an asterisk indicates termination codon.

In further detail with reference to Fig. 5, 230 DNA contains 741 nucleotides in open reading fram which may encode for a polypeptide of 247 amino acids. The thymopoietin peptide is near the N-terminus of the putative precursor protein. There are two translation initiation codons (ATG) in frame at positions 202 and 220, the last one just preceding the thymopoietin sequence. Thymopoietin peptide is encoded by nucleotides 223-369 and is identical to the published sequence of bovine thymopoietin II (Audhya, T. et al, Biochemistry loc cit) with three exceptions:

(a) the amino acid at position 43 is His as in TP-I and TP-III and not Ser as in TP-II;

(b) at positions 47 and 48, clone 230 encodes for Arg Asn instead of Leu Val, as published; and

(c) the amino acid at position 38 is Gln, whereas some publications report Glu, and some Gln, at this position. It is also of interest that the 230 DNA sequence and its corresponding peptide sequence are identical to the sequenced part of the bovine genomic clone 152B, including Glu 38 and His 43, in spite of the fact that the genomic and cDNA libraries were prepared from different calves.

Isolation and Characterization of Fetal Calf cDNA Clones Coding for Thymopoietin or Thymopoietin-like Peptides.

Fetal calf thymic cDNA library (Clontech, $1 \times 10^6$ plaque-forming units) was plated and screened by hybridization with cloned bovine cDNA fragment containing 126 bp coding for amino acids 1-42 of bovine thymopoietin. This fragment was produced by Sac I-Pst I digestion from one of the subclones used for the sequencing of cDNA clone 230. Experimental procedures were the same as described above for the screening of calf thymic epithelial cDNA library. Nine clones were isolated and analyzed by restriction digestion. Seven of these clones exhibited identical or overlapping restriction patterns, the other two (named 401 and 416), appeared different and were subjected to DNA sequencing in phagemid Bluescript vectors by the same approach as described above for clone 230.

DNA Sequence of Fetal Calf Thymopoietin Clone 416

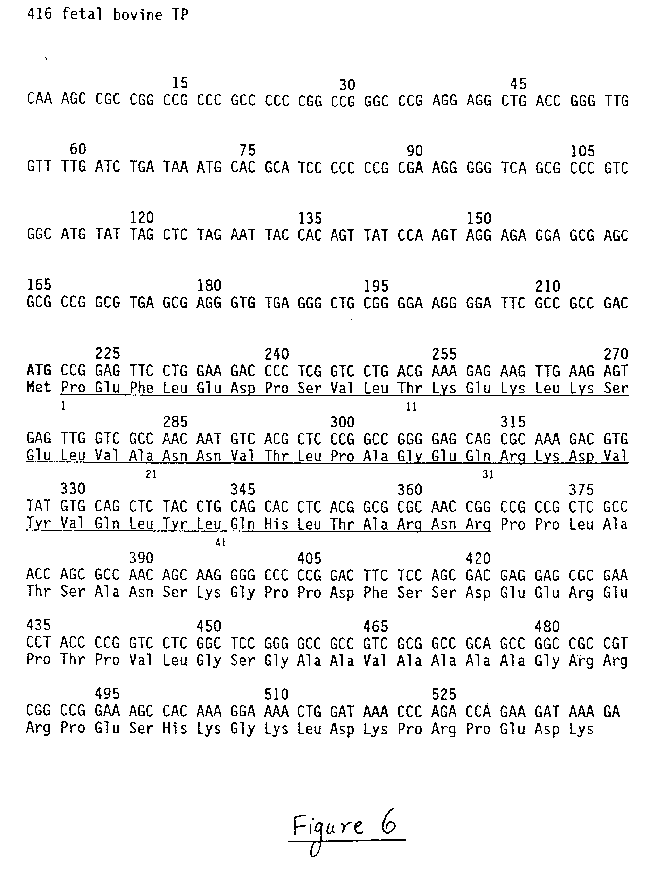

The DNA sequence of clone 416 and a peptide sequence deduced from it are shown in Fig. 6. The sequence according to the 49 amino acids of thymopoietin peptide is underlined. This sequence is identical to that coded by clone 230, including His 43, Arg 47 and Asn 48. Both clones differ in this respect from published Bovine TPII. This clone contains 539 bp, 323 of which are in an open reading frame, which corresponds to 108 amino acids and is incomplete as judged by lack of a translation termination codon at the 3' end. Nucleotides 204-493 of clone 416 are identical to nucleotides 207-496 of clone 230, nucleotides 178-345 of clone 416 are identical to nucleotides 158-325 of genomic subclone 152-13, and nucleotides 155-177 are partially identical to nucleotides 135-157 of the genomic subclone, while nucleotides 1-154 of the 5' untranslated region of 416 and nucleotides 494-539 of the open reading frame differ from either. Taking into consideration that the fetal clone 416 contains DNA sequences coding for the entire thymopoietin and a part of the thymopoietin precursor deduced from the cDNA isolated from suckling calves, we assume that clone 416 corresponds to a fetal variant of the thymopoietin precursor.

DNA Sequence of Calf Fetal Clone 401 Coding for a Thymopoietin-like Protein

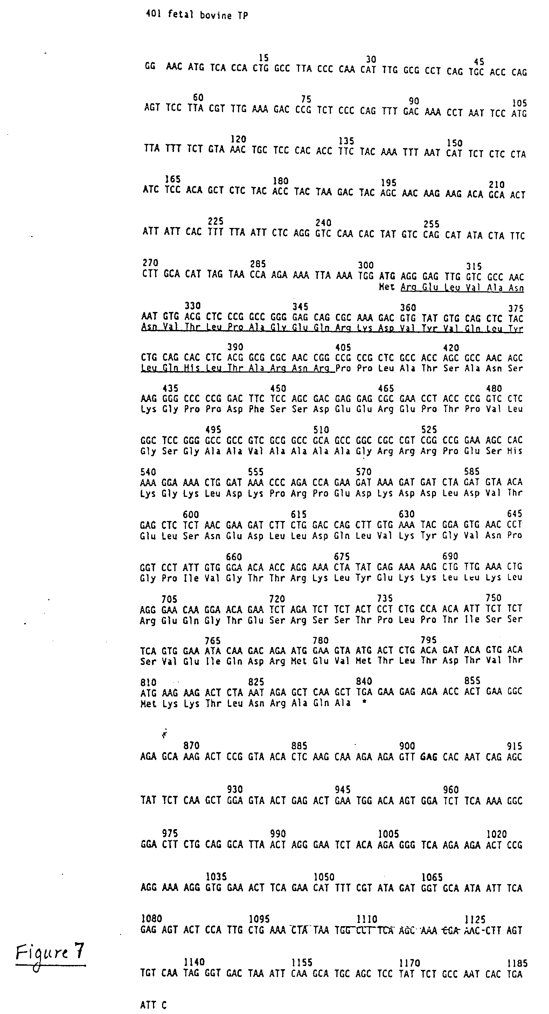

The DNA sequence of fetal calf cDNA clone 401 is shown in Fig. 7. The clone contains 1191 bp which includes 302 bp in the 5' untranslated region, 537 bp as an open reading frame (which can encode for a 179 amino acid precursor protein) and 351 bp in the 3' untranslated region. Underlined are 32 amino acids identical to amino acids 18-49 of bovine thymopoietin as deduced from clones 230 and 116. Nucleotides 309-531 of clone 401 are identical to nucleotides 274-496 of clone 230, and nucleotides 309-575 of clone 401 are identical to nucleotides 271-539 of clone 416 (including 45 nucleotides at the 3' end of clone 416 which differ from those of clone 230). The 5' untranslated part of clone 401 differs from the corresponding part of clones 230, 416 and 152-13. No conclusion can be drawn about the 3' part of clone 401, since it is longer and extends beyond a corresponding sequence of clone 416.

Taking into consideration the fact that all the four clones reported above, namely cDNA clone 230, cDNA clone 401, cDNA clone 416 and genomic clones 152B and 157A, share "blocs" of identical DNA sequences linked to the sequences that differ in different clones, we assume that the various cDNA clones may originate by differential splicing of the same gene, and that such differential splicing may be developmentally regulated, thus giving rise to a fetal-specific thymopoietin precursor.

While particular embodiments of the invention have been described hereinabove, it will be appreciated that the present invention is not limited to such embodiments, since many modifications and variations not requiring inventive input can be made by the skilled addressee. Accordingly, the essential concept, spirit and scope of the present invention will be better understood in the light of the claims which follow.

**Claims**

1.  A bovine thymopoietin or thymopoietin-like cDNA clone which contains a sequence corresponding to at least part of bovine thymopoietin mRNA by virtue of the fact that it includes at least the sequential nucleotides in the following structure corresponding to amino acids 18 to 49:

| 1 | 2 | 3 | 4 | 5 | 6 | 7 | 8 | 9 | 10 | 11 | 12 | 13 | 14 | 15 | 16 |
|---|---|---|---|---|---|---|---|---|----|----|----|----|----|----|----|
| CCG | GAG | TTC | CTG | GAA | GAC | CCC | TCG | GTC | CTG | ACG | AAA | GAG | AAG | TTG | AAG |

| 17 | 18 | 19 | 20 | 21 | 22 | 23 | 24 | 25 | 26 | 27 | 28 | 29 | 30 | 31 | 32 |
|----|----|----|----|----|----|----|----|----|----|----|----|----|----|----|----|
| AGT | GAG | TTG | GTC | GCC | AAC | AAT | GTG | ACG | CTC | CCG | GCC | GGG | GAG | CAG | CGC |

| 33 | 34 | 35 | 36 | 37 | 38 | 39 | 40 | 41 | 42 | 43 | 44 | 45 | 46 | 47 | 48 |
|----|----|----|----|----|----|----|----|----|----|----|----|----|----|----|----|
| AAA | GAC | GTG | TAT | GTG | CAG | CTC | TAC | CTG | CAG | CAC | CTC | ACG | GCG | CGC | AAC |

| 49 |
|----|
| CGG. |

2.  A cDNA clone according to claim 1, which includes the whole of the nucleotide sequence corresponding to amino acids 1 to 49 recited therein.

3.  A bovine thymopoietin cDNA clone, which is a cDNA clone as defined in claim 2, in which said nucleotide sequence corresponding to amino acids 1 to 49 is included in an open reading frame of 741 nucleotides.

4.  A partial bovine fetal thymopoietin cDNA clone, which is a cDNA clone as defined in claim 2, in which said nucleotide sequence corresponding to amino acids 1 to 49 is included in an open reading frame of 323 nucleotides, and wherein a 45 nucleotide sequence at the 3'-end of said open reading frame is different from that in the cDNA clone of claim 3.

5.  A bovine fetal thymopoietin-like cDNA clone, which is a cDNA clone as defined in claim 1, and which contains an open reading frame of 537 nucleotides corresponding to a 179 amino acid polypeptide of which the N-terminal part includes amino acids 18 to 49 of thymopoietin as recited in claim 1.

6. A bovine thymopoietin mRNA species having a nucleotide sequence corresponding to the nucleotide sequence of the cDNA clone according to any of claims 1 to 5.

7. The deduced protein sequence according to the translatable part of the mRNA species according to claim 6, and any bioactive cleavage fragment deduced therefrom.

8. Isolated and purified DNA clones which contain bovine thymopoietin gene sequence and include the sequence:

```
  1   2   3   4   5   6   7   8   9  10  11  12  13  14  15  16
CCG GAG TTC CTG GAA GAC CCC TCG GTC CTG ACG AAA GAG AAG TTG AAG

 17  18  19  20  21  22  23  24  25  26  27  28  29  30  31  32
AGT GAG TTG GTC GCC AAC AAT GTG ACG CTC CCG GCC GGG GAG CAG CGC

 33  34  35  36  37  38  39  40  41  42  43  44
AAA GAC GTG TAT GTG CAG CTC TAC CTG CAG CAC CTC.
```

9. A cDNA or DNA clone which is produced by hybridization from a clone according to any of claims 1 to 5.

10. A cDNA or DNA clone which is produced by hybridization from a clone as defined in claim 8.

FIG. 1

Figure 2

```
                15                      30                      45
C    TGC AGG GGC TTT TGT GTC CGC CTT TGG CTT GGC TTT GTG TGC GCG GGT TTC


                60                      75                      90                     105
CGC TGG GCT TCC AGG CGT CAG TCC CGG GCG GGA GCC GTG AGG CTC GGA GGC GGG


                120                     135                     150
AGC GCG GCC CCC CCT CCC CCG GGC GAG GAG CGA GCG CGC CGG CGT GAG CGG AGG


     165                     180                     195                     210
GTG TGA GGG CTG CGG GGA AGG GGA TTC GCC GCC GAG ATG CCG GAG TTC CTG GAA
     *   Gly Leu Arg Gly Arg Gly Phe Ala Ala Glu Met Pro Glu Phe Leu Glu
                                                             1


                225                     240                     255
GAC CCC TCG GTC CTG ACG AAA GAG AAG TTG AAG AGT GAG TTG GTC GCC AAC AAT
Asp Pro Ser Val Leu Thr Lys Glu Lys Leu Lys Ser Glu Leu Val Ala Asn Asn
                         11                                              21

270                     285                     300                     315
GTG ACG CTC CCG GCC GGG GAG CAG CGC AAA GAC GTG TAT GTG CAG CTC TAC CTG
Val Thr Leu Pro Ala Gly Glu Gln Arg Lys Asp Val Tyr Val Gln Leu Tyr Leu
                         31                                              41

CAG
Gln
```

Figure 3

open reading frame

bovine thymopoietin (TP)

λ ZAP

T3 prom
polylink. clon. site
5' EcoRI
EcoRI
TP
PstI
Ban I
Sac II
Xho I
EcoR V
Sac I
3' EcoRI
polylink. clon. site
T7 prom
λ ZAP

100 bp

Figure 4

230FULL - mRNA str confirmed on gel 31\10\89

```
                15              30              45
TCC AGG CAA GAG TAC TGG AGT GGA TTG CCA TTC CCT TCT CCA GAG GAA CTT CCA


        60              75              90              105
AAC CCA GGG ATC AAA CCC TGG TCT CCT GCA TCA TAG GCA GAT TCT TTA CCA TTT


            120             135             150
GAG CTA CAG GGA AGT CTA TAC TGT CTT TAG GAA TAT ATA AAA ATG AGC CTA GAG


165             180             195             210⌐
CGA ATA TTG TCA TAT TGA GAG AAT CTC ATT CTT TAA TAT ATG GAA TTC GCC GCC
                                                        Met Glu Phe Ala Ala


        225             240             255             270
GAG ATG CCG GAG TTC CTG GAA GAC CCC TCG GTC CTG ACG AAA GAG AAG TTG AAG
Glu Met Pro Glu Phe Leu Glu Asp Pro Ser Val Leu Thr Lys Glu Lys Leu Lys
1                       II
                285             300             315
AGT GAG TTG GTC GCC AAC AAT GTG ACG CTC CCG GCC GGG GAG CAG CGC AAA GAC
Ser Glu Leu Val Ala Asn Asn Val Thr Leu Pro Ala Gly Glu Gln Arg Lys Asp
                    2I                          3I
    330             345             360             375
GTG TAT GTG CAG CTC TAC CTG CAG CAC CTC ACG GCG CGC AAC CGG CCG CCG CTC
Val Tyr Val Gln Leu Tyr Leu Gln His Leu Thr Ala Arg Asn Arg Pro Pro Leu
                        4I
            390             405             420
GCC ACC AGC GCC AAC AGC AAG GGG CCC CCG GAC TTC TCC AGC GAC GAG GAG CGC
Ala Thr Ser Ala Asn Ser Lys Gly Pro Pro Asp Phe Ser Ser Asp Glu Glu Arg


435             450             465             480
GAA CCT ACC CCG GTC CTC GGC TCC GGG GCC GCC GTC GCG GCC GCA GCC GGC CGC
Glu Pro Thr Pro Val Leu Gly Ser Gly Ala Ala Val Ala Ala Ala Ala Gly Arg


            495             510             525             540
CGT CGG CCG GGT AAG GAC GCC CTG CCG GGG CCA CAA AGG CGG GCG TTG AGC AGT
Arg Arg Pro Gly Lys Asp Ala Leu Pro Gly Pro Gln Arg Arg Ala Leu Ser Ser


                555             570             585
GGG CGC CCC CTC GGG CCT CGG CGG CGG GGT GGG CCC CCC ACC CGC TCA GGG GGC
Gly Arg Pro Leu Gly Pro Arg Arg Arg Gly Gly Pro Pro Thr Arg Ser Gly Gly


600             615             630             645
TGG CCT CTA GGT CTC CGG ACG CCC GAC TCG GCC TCT TGG CCT CGG GCT CTG AGA
Trp Pro Leu Gly Leu Arg Thr Pro Asp Ser Ala Ser Trp Pro Arg Ala Leu Arg


            660             675             690
GGG GAG GGG CCG GAG TGG AGA GCC CCG AAG TTG GGG CCG CGG GGC GCG CGT CTT
Gly Glu Gly Pro Glu Trp Arg Ala Pro Lys Leu Gly Pro Arg Gly Ala Arg Leu
```

```
705             720             735             750
GGC CGC GGT GTG TCG TGC GGT GTG AAG TTA GCT TTG CTC TCG GGT TGC GCG CGG
Gly Arg Gly Val Ser Cys Gly Val Lys Leu Ala Leu Leu Ser Gly Cys Ala Arg


    765             780             795             810
ACA GCT CGG TGT CCC TGC ACC CCC GGC TGT CGG GAG TGG GTA GTG AGA CCG GGA
Thr Ala Arg Cys Pro Cys Thr Pro Gly Cys Arg Glu Trp Val Val Arg Pro Gly


            825             840             855
CTT CCC AGC CCT TTT CGA AAA GCC CTG AGA AGG GTT ATC TCG AAA CGG CTA CAG
Leu Pro Ser Pro Phe Arg Lys Ala Leu Arg Arg Val Ile Ser Lys Arg Leu Gln


870             885             900             915
GTT ACG CTC GAG CGT TTC CCC GCT TTA TTA ACC GAA ATG ACT GGT GAG CGG GTC
Val Thr Leu Glu Arg Phe Pro Ala Leu Leu Thr Glu Met Thr Gly Glu Arg Val


        930             945             960
TCA GAG CGC TCC CTG GAG GGC AAT TTG TGA CAT GTT GAT ATC TAA CCA AAT TTT
Ser Glu Arg Ser Leu Glu Gly Asn Leu *


975             990             1005            1020
GAA ATG AAA TTC CCG CCT TTT TAT GTT TCT TTG CAT AAG GAG GGC TTT TTG AAC


    1035            1050            1065            1080
GGT TTT TGT TTT TCT TGT TTG GGT TAC TCC TTC TAG CAC CAG GCT GTT TTT TGG


        1095            1110            1125
CGC GGC AGC ATT TCT CTA AAT TCA GCA AAC TTT TAA AAA TGT ATG TTG AAA GAC


    1140            1155            1170            1185
TAT CAG AGA CGC TCA AAT ATT TGA AAA TCC TAG GTA CTA GTG GTT TGG GAG GGT


        1200            1215            1230
TGG TTG TTT TGG TGT ATT TGC ATG AAG AAA GGA GAG CCT TTG GAA TTA GGA GAA


1245            1260            1275            1290
ATG TTT ATT TCG TGA CAT TTC TGC TTT GTT GGT GTT GCA GTT TGA AAT ATT TAA


    1305
TCC CGA ATT C
```

Figure 5

416 fetal bovine TP

```
                  15                    30                    45
CAA AGC CGC CGG CCG CCC GCC CCC CGG CCG GGC CCG AGG AGG CTG ACC GGG TTG


                  60                    75                    90                   105
GTT TTG ATC TGA TAA ATG CAC GCA TCC CCC CCG CGA AGG GGG TCA GCG CCC GTC


                 120                   135                   150
GGC ATG TAT TAG CTC TAG AAT TAC CAC AGT TAT CCA AGT AGG AGA GGA GCG AGC


165                   180                   195                   210
GCG CCG GCG TGA GCG AGG GTG TGA GGG CTG CGG GGA AGG GGA TTC GCC GCC GAC


                 225                   240                   255                   270
ATG CCG GAG TTC CTG GAA GAC CCC TCG GTC CTG ACG AAA GAG AAG TTG AAG AGT
Met Pro Glu Phe Leu Glu Asp Pro Ser Val Leu Thr Lys Glu Lys Leu Lys Ser
    1                                               11
                 285                   300                   315
GAG TTG GTC GCC AAC AAT GTC ACG CTC CCG GCC GGG GAG CAG CGC AAA GAC GTG
Glu Leu Val Ala Asn Asn Val Thr Leu Pro Ala Gly Glu Gln Arg Lys Asp Val
                 21                                              31
330                   345                   360                   375
TAT GTG CAG CTC TAC CTG CAG CAC CTC ACG GCG CGC AAC CGG CCG CCG CTC GCC
Tyr Val Gln Leu Tyr Leu Gln His Leu Thr Ala Arg Asn Arg Pro Pro Leu Ala
                 41
                 390                   405                   420
ACC AGC GCC AAC AGC AAG GGG CCC CCG GAC TTC TCC AGC GAC GAG GAG CGC GAA
Thr Ser Ala Asn Ser Lys Gly Pro Pro Asp Phe Ser Ser Asp Glu Glu Arg Glu

435                   450                   465                   480
CCT ACC CCG GTC CTC GGC TCC GGG GCC GCC GTC GCG GCC GCA GCC GGC CGC CGT
Pro Thr Pro Val Leu Gly Ser Gly Ala Ala Val Ala Ala Ala Ala Gly Arg Arg

                 495                   510                   525
CGG CCG GAA AGC CAC AAA GGA AAA CTG GAT AAA CCC AGA CCA GAA GAT AAA GA
Arg Pro Glu Ser His Lys Gly Lys Leu Asp Lys Pro Arg Pro Glu Asp Lys
```

Figure 6

401 fetal bovine TP

```
          15                   30                   45
GG  AAC ATG TCA CCA CTG GCC TTA CCC CAA CAT TTG GCG CCT CAG TGC ACC CAG

      60                   75                   90                   105
AGT TCC TTA CGT TTG AAA GAC CCG TCT CCC CAG TTT GAC AAA CCT AAT TCC ATG

          120                  135                  150
TTA TTT TCT GTA AAC TGC TCC CAC ACC TTC TAC AAA TTT AAT CAT TCT CTC CTA

    165                  180                  195                  210
ATC TCC ACA GCT CTC TAC ACC TAC TAA GAC TAC AGC AAC AAG AAG ACA GCA ACT

              225                  240                  255
ATT ATT CAC TTT TTA ATT CTC AGG GTC CAA CAC TAT GTC CAG CAT ATA CTA TTC

270                  285                  300                  315
CTT GCA CAT TAG TAA CCA AGA AAA TTA AAA TGG ATG AGG GAG TTG GTC GCC AAC
                                            Met Arg Glu Leu Val Ala Asn

        330                  345                  360                  375
AAT GTG ACG CTC CCG GCC GGG GAG CAG CGC AAA GAC GTG TAT GTG CAG CTC TAC
Asn Val Thr Leu Pro Ala Gly Glu Gln Arg Lys Asp Val Tyr Val Gln Leu Tyr

            390                  405                  420
CTG CAG CAC CTC ACG GCG CGC AAC CGG CCG CCG CTC GCC ACC AGC GCC AAC AGC
Leu Gln His Leu Thr Ala Arg Asn Arg Pro Pro Leu Ala Thr Ser Ala Asn Ser

    435                  450                  465                  480
AAG GGG CCC CCG GAC TTC TCC AGC GAC GAG GAG CGC GAA CCT ACC CCG GTC CTC
Lys Gly Pro Pro Asp Phe Ser Ser Asp Glu Glu Arg Glu Pro Thr Pro Val Leu

            495                  510                  525
GGC TCC GGG GCC GCC GTC GCG GCC GCA GCC GGC CGC CGT CGG CCG GAA AGC CAC
Gly Ser Gly Ala Ala Val Ala Ala Ala Gly Arg Arg Arg Pro Glu Ser His

540                  555                  570                  585
AAA GGA AAA CTG GAT AAA CCC AGA CCA GAA GAT AAA GAT GAT CTA GAT GTA ACA
Lys Gly Lys Leu Asp Lys Pro Arg Pro Glu Asp Lys Asp Asp Leu Asp Val Thr

        600                  615                  630                  645
GAG CTC TCT AAC GAA GAT CTT CTG GAC CAG CTT GTG AAA TAC GGA GTG AAC CCT
Glu Leu Ser Asn Glu Asp Leu Leu Asp Gln Leu Val Lys Tyr Gly Val Asn Pro

            660                  675                  690
GGT CCT ATT GTG GGA ACA ACC AGG AAA CTA TAT GAG AAA AAG CTG TTG AAA CTG
Gly Pro Ile Val Gly Thr Thr Arg Lys Leu Tyr Glu Lys Lys Leu Leu Lys Leu

    705                  720                  735                  750
AGG GAA CAA GGA ACA GAA TCT AGA TCT TCT ACT CCT CTG CCA ACA ATT TCT TCT
Arg Glu Gln Gly Thr Glu Ser Arg Ser Ser Thr Pro Leu Pro Thr Ile Ser Ser

        765                  780                  795
TCA GTG GAA ATA CAA GAC AGA ATG GAA GTA ATG ACT CTG ACA GAT ACA GTG ACA
Ser Val Glu Ile Gln Asp Arg Met Glu Val Met Thr Leu Thr Asp Thr Val Thr

810                  825                  840                  855
ATG AAG AAG ACT CTA AAT AGA GCT CAA GCT TGA GAA GAG AGA ACC ACT GAA GGC
Met Lys Lys Thr Leu Asn Arg Ala Gln Ala  *

        870                  885                  900                  915
AGA GCA AAG ACT CCG GTA ACA CTC AAG CAA AGA AGA GTT GAG CAC AAT CAG AGC

            930                  945                  960
TAT TCT CAA GCT GGA GTA ACT GAG ACT GAA TGG ACA AGT GGA TCT TCA AAA GGC

    975                  990                  1005                 1020
GGA CTT CTG CAG GCA TTA ACT AGG GAA TCT ACA AGA GGG TCA AGA AGA ACT CCG

            1035                 1050                 1065
AGG AAA AGG GTG GAA ACT TCA GAA CAT TTT CGT ATA GAT GGT GCA ATA ATT TCA

1080                 1095                 1110                 1125
GAG AGT ACT CCA TTG CTG AAA CTA TAA TGG CCT TCA AGC AAA GGA AAC CTT AGT

        1140                 1155                 1170                 1185
TGT CAA TAG GGT GAC TAA ATT CAA GCA TGC AGC TCC TAT TCT GCC AAT CAC TGA

ATT C
```

Figure 7

European Patent
Office

EUROPEAN SEARCH REPORT

Application Number

EP    92 30 0671
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 077 949 (G. GOLDSTEIN)<br>* Whole document *<br>--- | 1-10 | C12N15/16<br>C07K15/00 |
| Y | JOURNAL CELLULAR BIOCHEMISTRY SUPPLEMENT<br>vol. 0, no. 10A, 1986, NEW YORK, US<br>page 77;<br>L.K. JOLLIFFE ET AL.: 'Partial characterization of the genes encoding bovine and human immunoregulatory peptide hormones'<br>* Whole article *<br>--- | 1-10 | |
| A | BIOTECHNOLOGY ABSTRACTS. DERWENT PUBLICATIONS, LTD. LONDON. ABSTRACT NO 92-00795<br>M. RACK ET AL.: 'Synthesis of thymopoietin - artificial gene cloning and expression in Escherichia coli inclusion body; fusion protein cleavage and protein renaturation.'<br>& DECHEMA Biotechnol. Conf. 1990, vol. 4, pt. A, p 411-413<br>* Abstract *<br>--- | 1,6-10 | |
| Y | ZEITSCHRIFT FÜR CHEMIE<br>vol. 30, January 1990, LEIPZIG, GERMANY<br>pages 252 - 253;<br>W.-V. MEISTER ET AL.: 'Synthesen von Spleno- und Thymopentin-Gensondensequenzen'<br>* Whole article *<br>--- | 1-10 | TECHNICAL FIELDS SEARCHED (Int. Cl.5)<br><br>C07K |
| D,Y | BIOCHEMISTRY.<br>vol. 20, no. 21, 13 October 1981, EASTON, PA US<br>pages 6195 - 6200;<br>T. AUDHYA ET AL.: 'Complete amino acid sequences of bovine thymopoietins I, II and III : closely homologous polypeptides'<br>* Whole article *<br>---<br>-/-- | 1-10 | |

The present search report has been drawn up for all claims

| Place of search | Date of completion of the search | Examiner |
|---|---|---|
| BERLIN | 19 MAY 1992 | JULIA P. |

EPO FORM 1503 03.82 (P0401)

European Patent

Office

**EUROPEAN SEARCH REPORT**

Application Number

EP    92 30 0671
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| T | IMMUNOLOGY LETTERS vol. 31, no. 3, 15 February 1992, NL pages 301 - 310; D. ZEVIN-SONKIN ET AL.: 'Molecular cloning of the bovine thymopoietin gene and its expression in different calf tissues : evidence for a predominant expression in thymocytes' * Whole article * | 1-10 | |
| | ----- | | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 19 MAY 1992 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P0401)